# FASCICULE DE BREVET EUROPEEN

(11) **EP 1 250 665 B1**
(45) Date de publication et mention de la délivrance du brevet: **24.09.2003**
(21) Numéro de dépôt: 01907638.9
(22) Date de dépôt: 19.01.2001
(51) Int. Cl.: G06F 17/30

(54) **PROCEDE ET SYSTEME POUR GERER ET UTILISER UN FICHIER D'ITEMS DANS UN ESPACE D'INFORMATIONS, NOTAMMENT D'INFORMATIONS MEDICALES, ET APPLICATION A DES SYSTEMES D'INFORMATION**
VERFAHREN UND SYSTEM ZUR VERWALTUNG UND BENUTZUNG VON ARTIKELDATEIEN IN EINEM INFORMATIONSRAUM, INSBESONDERE MEDIZINISCHE INFORMATION, UND VERWENDUNG IN INFORMATIONSSYSTEMEN
METHOD AND SYSTEM FOR MANAGING AND USING AN ITEM FILE IN AN INFORMATION SPACE, IN PARTICULAR MEDICAL DATA, AND APPLICATION TO INFORMATION SYSTEM

(30) Priorité: 20.01.2000 WO PCT/FR00/00131
(43) Date de publication de la demande: 23.10.2002
(73) Titulaire: IMS Health, 92882 Nanterre CTC Cédex 9 (FR)
(72) Inventeur: LAMBERT, Bruno, F-92420 Vaucresson (FR)
(74) Mandataire: Pontet, Bernard
(86) Numéro de dépôt international: FR0100173
(87) Numéro de publication internationale: WO01053987

(56) Documents cités:
- WO-A-96/27161
- US-A- 5 678 038
- US-A- 5 812 995
- US-A- 5 930 788
- SEETHARAMA S: "Towards establishing concordance among medical classification systems" TKE '90: TERMINOLOGY AND KNOWLEDGE ENGINEERING. PROCEEDINGS SECOND INTERNATIONAL CONGRESS, TRIER, GERMANY, 2-4 OCT. 1990, pages 405-408 vol.2, XP000972717 1990, Frankfurt, Germany, Indeks Verlag, Germany

## Description

La présente invention concerne un procédé pour gérer et utiliser un fichier d'items dans un espace d'informations, notamment d'informations médicales. Elle vise également un système mettant en oeuvre ce procédé, ainsi que son application notamment à des systèmes d'information.

Dans le domaine de l'information scientifique et plus particulièrement médicale, les bases de données sont devenues les outils et vecteurs essentiels de l'accès aux informations. Ces informations font nécessairement l'objet de classifications et de codifications permettant une identification et un accès automatisé aux informations recherchées.

Or, les utilisateurs de ces bases qui la plupart du temps ne sont pas des spécialistes des systèmes de classification et de codification, se heurtent souvent à des difficultés d'accès aux informations du fait de la méconnaissance des mécanismes d'arborescence attachés à ces systèmes. Il en découle généralement une perte de temps et d'efficacité.

Ce problème est aggravé lorsqu'il s'agit d'un domaine scientifique ou technique dans lequel coexistent plusieurs systèmes de classification. On peut notamment citer le cas des bases de données médicales qui doivent prendre en compte actuellement une transition entre la classification OMS (CIM9) et la nouvelle classification OMS (CIM10). On peut aussi citer le domaine des brevets dans lequel coexistent au moins deux systèmes de classification, à savoir le système international (OMPI) de classification des brevets, et le système américain de classification.

Des classifications d'objet même proche (par exemple médicales) n'ont en général pas des relations réciproques simples entre les codes de chacune. Cette situation est très fréquente et pose des problèmes pour la continuité d'analyse des observations liées au moment d'une transition de nomenclature, avec de plus un fort risque, ou même une quasi certitude, de perte de données antérieures, et celui de générer des artefacts graves dans la série des données. La « finesse » de cette transition est pourtant souvent requise en complément ou est même indispensable dans de nombreux domaines.

Ce besoin important et fréquent est même souvent évoqué comme particulièrement délicat et non résolu. Par exemple, le document XP002097913 [Frutiger : « Multipurpose computerized record for clinical research, institutional management and government health data administration » Role of informatics in health data coding and classification systems, 26 - 28 Septembre 1984 Otawa Canada] précise que le problème de relation (transpositions ou transcodage) est non résolu et le présente même comme l'un des défis les plus difficiles dans ce domaine.

En effet, la relation entre codes de diverses classifications est souvent de nature « 1 » vers « n » et la transposition des données d'une classification vers une autre est donc très délicate ou même impossible. La connaissance de l'acception de ces codes n'est ici pas suffisante pour établir le choix du lien pertinent.

Il n'existe pas à ce jour de solution correcte à ce problème. Au mieux, même dans le cas d'une transition de version de classification, une table de correspondance peut éventuellement être proposée. Mais une table de correspondance ne peut résoudre le problème des relations « 1 » vers « n » et de l'éclatement d'un code vers ceux d'une autre classification, car elle n'offre au mieux que la liste des possibilités multiples. La décision reste alors à prendre parmi « n » possibilités, sans disposer pour autant d'éléments objectifs qui permettent de légitimer le choix à effectuer. Même dans une situation de relation « 1 » vers « 1 », l'obtention du « bon » code dépend là aussi de la qualité du codeur et de la constance de sa décision.

Il est par ailleurs important de distinguer le contexte local du contexte catégoriel et de remarquer que les apparentes solutions existantes ne sont en aucun cas adaptées au contexte local des données observées.

Le but de l'invention est de remédier à ces inconvénients en proposant un procédé de génération et d'utilisation d'un fichier d'items qui permette à un utilisateur de ce fichier d'évoluer dans un univers sémantique et un langage, en particulier le langage naturel, qui lui sont familiers sans lui imposer de connaître et d'utiliser des classifications et codifications.

On atteint les objectifs précités avec un procédé pour générer et utiliser un fichier d'items dans un espace d'informations, notamment d'informations médicales, stockées dans au moins une base de données et pouvant être classées selon une pluralité de systèmes de classification, ce fichier d'items étant généré par accumulation d'items rédigés dans un langage dont le langage naturel. Ce procédé comprend, pour chaque item au sein de ce fichier d'items, une attribution, en plus de ses caractéristiques propres, de plusieurs codes de classification appartenant chacun à un système de classification, de sorte que les codes de classification ainsi attribués procurent des liens avec d'autres fonctionnalités ou bases de données.

Suivant l'invention, ce procédé comprend en outre les étapes consistant à :
- affecter à chaque item la propriété de constituer la plus petite partie commune entre différents codes de classification attribués,
- attribuer à chaque item constituant la plus petite partie commune entre les différents codes de classification attribués audit item, un code unique qui lui est propre, et
- détecter ceux des items issus du fichier d'items, qui sont liés à « n » codes de « n » systèmes de classification et qui sont le lieu d'une relation bijective entre les codes de ces « n » systèmes de classification dont lesdits items sont l'intersection.

La solution novatrice apportée par le procédé selon l'invention repose ainsi sur la combinaison d'un fichier d'items avec leurs liens individuels aux codes des classifications associées, sur la connaissance des items du fichier d'items qui sont liés à un code d'une classification, et sur la capacité de connaître les items issus du fichier d'items qui sont sous-jacents d'un code et qui sont le lieu d'une relation « 1 »-« 1 » (et non plus « 1 » vers « n ») entre les codes des deux classifications ou versions de classifications.

Les éléments du fichier d'items constituent chacun un niveau intermédiaire le plus fin, désigné dans la suite par l'acronyme « IBL » ('In Between Level'), qui représente la plus petite partie commune, intermédiaire entre des codes d'un premier système de classification vers un code d'un i-ème code de classification.

L'utilisation de la combinaison de ces informations et de leurs propriétés permet de révéler et de quantifier des passerelles (bridges) entre codes de classifications. Ceci est particulièrement utile dans le cas où ces classifications ne disposent pas seulement de relations de code (CL1) à code (Cli) de type « 1 » vers « 1 », mais aussi de type « 1 » vers « n ».

Dans un mode de mise en oeuvre particulièrement avantageux du procédé selon l'invention, celui-ci est en outre agencé pour procurer un transcodage entre un premier système de classification et un second système de classification parmi la pluralité de systèmes de classification via le fichier d'items généré avec ce procédé.

Ce transcodage peut notamment prendre en compte des caractéristiques des items, notamment des caractéristiques de fréquence, accessibles via le code unique ou via l'item.

On peut prévoir que des items du fichier d'items revêtent la nature de terme ou locution de type nomenclature, chacun de ces items constituant un élément commun d'intersection de l'acception de deux codes de deux classifications différentes.

Le procédé selon l'invention peut en outre comprendre un traitement de caractéristiques, notamment de fréquence, d'items du fichier d'items pour pondérer des observations réelles des items constituant la plus petite partie commune entre plusieurs systèmes de classification.

Le code unique attaché à chaque item au sein du fichier d'items est de préférence, mais non limitativement, attribué séquentiellement. Ce code unique est de préférence agencé pour procurer lui aussi un lien simple avec d'autres caractéristiques de l'item, notamment des caractéristiques d'observations antérieures.

Le procédé selon l'invention peut également comprendre une sélection, au sein du fichier d'items, d'items répondant aux attentes d'un utilisateur, de façon à constituer un fichier résultat extrait du fichier d'items et pourvu des mêmes caractéristiques de lien avec des bases et fonctionnalités extérieures. Il peut aussi être agencé pour générer, pour chaque ligne du fichier d'items, un libellé pouvant être rédigé en langage naturel et constituant l'item, ainsi qu'un code unique attaché à ce libellé et plusieurs codes issus de plusieurs classifications.

Suivant un autre aspect de l'invention, il est proposé un système pour gérer et utiliser un fichier d'items dans un espace d'informations, notamment d'informations médicales, stockées dans au moins une base de données et pouvant être classées selon une pluralité de systèmes de classification, mettant en oeuvre le procédé selon l'invention, ce système comprenant:
- des moyens pour accumuler, au sein de ce fichier d'items, des items rédigés dans un langage tel que le langage naturel par des utilisateurs de cette base de données, et des moyens pour attribuer à chaque item au sein dudit fichier d'items plusieurs codes de classification appartenant chacun à un système de classification parmi la pluralité de systèmes de classification, de sorte que les codes de classification ainsi attribués procurent des liens avec d'autres fonctionnalités ou bases de données.

Ce système de gestion et d'utilisation de fichier d'items est caractérisé en ce qu'il comprend en outre :
- des moyens pour attribuer à chaque item constituant la plus petite partie commune entre les différents codes de classification attribués audit item, un code unique qui lui est propre,
- des moyens pour associer à chaque code de classification appartenant à un système de classification, les items auxquels ledit code de classification a été attribué, et
- des moyens pour identifier ceux des items issus du fichier d'items, qui sont liés à « n » codes de classification de « n » systèmes de classification et sont le lieu d'une relation bijective entre les codes de ces « n » systèmes de classification dont lesdits items sont l'intersection.

Le système selon l'invention peut en outre avantageusement comprendre des moyens pour réaliser un transcodage entre un premier système de classification et un second système de classification parmi la pluralité de systèmes de classification via le fichier de codification généré avec ce procédé.

Le procédé et le système de gestion et d'utilisation d'un fichier d'items selon l'invention peuvent être mis en oeuvre dans un système d'information médicale, chaque item consistant alors en un libellé de maladies, diagnostics ou raisons de consultation médicale.

Plus généralement, ils peuvent être appliqués dans un système d'information, notamment un système d'information médicale, le fichier d'items procurant notamment un accès à des bases de données et fonctionnalités qui utilisent
ou contiennent l'une au moins des classifications utilisées dans ce fichier d'items ou dans un fichier résultant d'un choix effectué au sein dudit fichier d'items.

L'application du procédé selon l'invention peut permettre :
- une transition (transcodage) entre classifications et/ou systèmes liés, par exemple entre des classifications médicales telles que la classification CIM et la classification DSM ;
- une limitation ou même une disparition des artefacts de la transition de classification induits par des éclatements ou regroupements de codes quand cette transition était tentée sans connaissance plus fine ;
- une continuité de séries temporelles d'observations dans un contexte de changement de version de la (ou des) classification(s), qui permet de générer un historique de données antérieurement collectées, converties selon la nouvelle classification. On peut citer par exemple les différentes versions consécutives (CIM9-CIM10) de classification des maladies émises par l'Organisation Mondiale de la Santé (OMS);
- une mise en oeuvre des transitions dont la finesse peut être spécifique des observations spécifiques du pays ou du contexte local de cette transition, ce qui revient à la création de passerelles générales, mais dont l'application peut être modulée par des observations locales (régionales ou nationales) et/ou catégorielles (médecin généralistes et/ou médecins spécialistes, ...).

On peut ainsi avec le procédé selon l'invention éviter la création d'une passerelle (bridge) de transition générale qui ne tiendrait pas compte des particularités locales. Cette propriété repose sur la pondération par les fréquences des observations réelles et des items constituant la « plus petite partie commune » entre ces « n » systèmes de classification.

Le procédé selon l'invention permet en outre, dans les cas où les données ne sont connues que sous la forme d'un code d'une classification antérieure, de les réaffecter selon une classification plus récente ou différente, en utilisant les résultats de tables de correspondance avec pondération de code à code de l'un vers l'autre de ces systèmes de classification.

On peut citer, à titre d'exemple non limitatif, le cas de données collectées in fine sous la seule information du code CIM9 de l'OMS et dont la conservation est utile, mais dont la conversion en structure CIM10 est indispensable. Ceci permet la re-génération de données historiques en cas de contexte délicat, limitant à minima la génération d'artefacts.

Il est à noter que chaque libellé est lui-même unique dans le fichier offert au choix des utilisateurs. Le code unique n'est que l'un des qualifiants, parmi d'autres, de ce libellé unique. Ce code unique permet un lien simple avec d'autres caractéristiques dont, par exemple, des caractéristiques d'observations antérieures qui qualifient l'item par des notions de fréquence et d'origine.

Il est important de remarquer que le fichier de codification ainsi obtenu est issu d'un travail de collecte et de traitement en amont, puis est offert au choix d'un utilisateur. Celui-ci choisit au sein de ce fichier de codification un ou plusieurs items répondant à son attente qui sont stockés dans un fichier résultant du choix de l'utilisateur.

On peut en outre avantageusement utiliser le procédé selon l'invention pour procurer un transcodage entre un premier système de classification et un second système de classification parmi la pluralité de systèmes de classification via le fichier de codification généré avec ce procédé.

Dans une forme pratique d'implémentation du procédé selon l'invention, celui-ci est agencé pour générer, pour chaque ligne du fichier de codification, un libellé rédigé en langage naturel et constituant l'item, un code unique attaché à ce libellé, et un ou plusieurs codes d'une ou plusieurs classifications.

D'autres particularités et avantages de l'invention apparaîtront encore dans la description ci-après. Aux dessins annexés donnés à titre d'exemples non limitatifs:
- la figure 1 illustre les étapes essentielles du procédé de gestion d'un fichier d'items selon l'invention;
- la figure 2 illustre un mode d'utilisation d'un fichier d'items généré avec le procédé selon l'invention, avec accès par liens dynamiques à des fonctionnalités extérieures; et
- la figure 3 illustre une opération de transcodage entre deux classifications réalisée avec le procédé selon l'invention.

Il est en premier lieu important de préciser ce que représente un fichier d'items dans le contexte du procédé et du système selon la présente invention. Il s'agit d'un fichier dans lequel sont accumulés des items principalement composés de termes en langage naturel et d'abréviations usuelles. A chaque item qui possède la propriété de « plus petite partie commune », sont attribués un ou plusieurs codes appartenant à plusieurs systèmes de classification et liés éventuellement à des sources de données et des fonctionnalités, directement ou indirectement.

En réciproque, un code d'une classification cible peut correspondre ou être lié à plusieurs items d'un fichier d'items, qui lui sont affectés. Chaque item d'un fichier d'items peut bénéficier d'un identifiant unique qui lui est propre. Cet identifiant unique peut être fixe et continu et peut par exemple être généré séquentiellement.

Chaque item peut être qualifié par exemple par des résultats d'observations (fréquences, ...) puis par son code unique DFCC d'identification qui lui est propre.

La mise en oeuvre du procédé selon l'invention contribue à une simplification des systèmes d'information, à une meilleure transparence en matière d'accès aux information et de transcodage et à de nombreux bénéfices induits.

En premier lieu, les items peuvent être librement rédigés en langage naturel, ce qui procure une grande facilité d'emploi par les utilisateurs, par exemple des médecins. Par ailleurs, le procédé selon l'invention permet l'accès à des fonctions évoluées sans action volontaire directe. Par exemple, à partir d'une saisie en langage naturel de diagnostics (ou symptômes ou syndromes) médicaux, le médecin obtient sans difficulté la génération sous-jacente d'un code d'une classification dont l'export est par exemple requis pour raison administrative.

Le médecin utilisateur de ce procédé peut alors consacrer l'essentiel de son temps à la médecine lors de l'entretien avec le malade, en effectuant entre autre le simple choix de ses termes diagnostiques, symptômes ou syndromes. Il n'est donc pas contraint de se transformer en codeur ou spécialiste de la codification, mais peut, sans perte de temps, répondre à ses obligation légales complémentaires.

Le médecin utilisateur d'un fichier d'items généré par mise en oeuvre du procédé selon l'invention peut en outre bénéficier de services induits par l'usage d'éléments communs tels qu'un code de diagnostic qui, d'une part, résulte du seul choix d'un item IBL du fichiers d'items FC et, d'autre part, est un code de pathologie également présent dans une base de produits qu'il qualifie positivement ou négativement en terme d'indication ou au contraire de contre indication.

La mise en oeuvre du procédé de gestion et d'utilisation d'un fichier d'items procure également des avantages significatifs en termes de reproductibilité et de constante des résultats de codification. Ainsi, pour un même contexte, le procédé de gestion et d'utilisation va conduire à un même résultat en ce qui concerne le code de la classification à générer in fine, indépendamment de la compétence des utilisateurs quand bien même ces utilisateurs seraient de niveaux de compétence différents.

Cette constance dans les résultats de codification procurés par le procédé selon l'invention est particulièrement avantageuse pour un utilisateur tel qu'un médecin, tant pour son utilisation propre dans sa pratique médicale en assurant la cohérence de ses décisions, que pour satisfaire efficacement et de manière transparente des obligations légales en matière de codification des pathologies et de transmission de codes à des tiers.

En outre, le fait que le résultat de codification obtenu par mise en oeuvre du procédé selon l'invention ne dépend pas de la compétence des multiples utilisateurs de fichiers d'items en matière de codification ou de l'incidence économique du code choisi sur le remboursement ou le paiement d'un acte, mais qu'il ne dépend que de la seule nature de l'item sélectionné en entrée, peut contribuer à limiter le risque de détournement volontaire ou fortuit du choix du code vers un code « plus rentable » mais erroné.

De plus, le procédé selon l'invention procure à l'utilisateur une facilité de mise à jour et de correction du pointage sur un code de classification. Ainsi, on peut prévoir la constitution d'un fichier d'observation, sous partie qualifiée de fichier d'items pourvu de codes DFCC et de fréquences, qui peut avoir des éléments dont les liens avec des codes de classification peuvent être remis en cause.

La correction éventuelle de certains de ces liens (par exemple, « IBL(a) lié au Code(1) de CL(i) » remplacé par « IBL(a) lié au Code(n) de Cli ») ne remet pas en cause le sous-ensemble constitué des items IBL et de leurs caractéristiques propres.

La visualisation correspondante selon la même classification (CLi), après correction des liens entre item IBL et code de la classification, permet de conserver l'ensemble des données du sous-ensemble et de les observer selon la nouvelle version corrigée de la relation.

Ceci ouvre la possibilité d'effectuer des corrections de la matière de base (fichier FC des items IBL en langage naturel ou autre) pour leurs relations avec les classifications liées. En effet, il y a là une possibilité d'effectuer des corrections des codes liés à chaque élément individuel sans remettre en cause la nature des items qui avaient étés initialement retenus (fichiers des items en langage naturel ou autre). Ceci est un corollaire de la possibilité de relation avec « n » classifications dont les versions peuvent évoluer.

Les applications du procédé selon l'invention sont importantes en ce qu'il est possible de disjoindre la notion de relevé ou de choix d'item IBL d'une part, et d'autre part de mettre à jour en différé les liens qui qualifient ces items. On peut ainsi effectuer un recueil (choix qualifié d'items IBL) anticipant la connaissance de la structure de classification selon laquelle il sera analysé ultérieurement. Le code propre DFCC de l'item IBL, ou l'item IBL lui même, est l'un des items qu'il suffit de conserver, lui même étant invariant au cours du temps.

La mise à jour des liens du fichier d'items peut être réalisée localement ou faire l'objet d'une mise à jour partagée par de nombreux utilisateurs du fichier d'items. On envisage ici la constitution d'un fichier FC, rendu vivant par ses mises à jour régulières, tant pour les items qui le constituent par exemple par ajout de novations issues éventuellement des utilisateurs eux mêmes, soit aussi par ajout de liens à des codes de classifications nouvelles ou de modifications (corrections de liens) avec des classifications déjà présentes.

Il en résulte une possibilité d'uniformisation des décisions émises par les utilisateurs du procédé selon l'invention, du fait de la cohérence des bases de choix. Cette uniformisation peut être mise en oeuvre dans le domaine médical, avec la mise à disposition d'une base de fichiers d'items qui résulte d'une accumulation des items communément recherchés pour décrire les pathologies et dont les liens aux classifications sont l'objet d'un consensus lui aussi régulièrement mis à jour par des autorités compétentes, par exemple par un collège médical représentatif et compétent. L'incidence bénéfique de cette uniformisation est importante dans ce domaine où l'informatisation est naissante et où la codification devient une exigence parfois légale et aussi personnelle.

Le procédé selon l'invention permet l'activation de fonctionnalités sous-jacentes, au nombre desquelles la fonctionnalité de signalement d'incohérence ou de danger.

A titre d'exemple non limitatif, un médecin ayant pu décrire de façon simple le contexte de pathologies de son patient par des choix d'items IBL au sein du fichier FC, génère en transparence les codes correspondants par exemple des codes de la classification CIM10 de l'OMS. Il prescrit par ailleurs des produits de traitement.

Ces produits sont décrits dans une base accessible directement à partir du fichier FC, avec en outre des informations sur leurs indications en termes de pathologies et de contre-indication pour certaines pathologies.

Ceci ouvre la possibilité de qualifier la relation entre, d'une part, la consultation du médecin (produits prescrits et contextes pathologiques) et, d'autre part, les caractéristiques d'éventuelles contre-indications de ces produits ou molécules retenus en prescription des pathologies retenues. Ceci permet d'apporter au médecin utilisateur une sécurité essentielle à l'égard de son patient lors de son exercice professionnel.

Par ailleurs, à partir de la connaissance des sous-parties d'un code (Cli) en ses « plus petits éléments », il est possible d'effectuer l'analyse de sous-ensembles cohérents. Ces sous-ensembles sont normalement invisibles lors d'une utilisation classique de la classification. Ils sont même en général perdus car, s'ils constituent de fait une étape de la décision, ils ne sont en général pas retenus comme le code final. Le processus présenté ici repose sur un seul choix initial qui lui, retient au contraire l'item IBL, et donc le code Cli qui lui est associé.

Ces sous-ensembles peuvent cependant correspondre à des segmentations pertinentes qui ne seraient pas accessibles par la seule connaissance de ce code ni même de ses caractéristiques (présence, fréquence).

Le procédé selon l'invention peut également prendre en compte un traitement des données selon une évolution prévisible de la classification, avant qu'elle ne soit mise en oeuvre par une révision officielle. On peut donc par exemple et de façon non limitative devancer des améliorations de l'évolution d'une classification tout en conservant la capacité à communiquer selon son standard du moment, ce qui introduit la notion d'évolution intermédiaire d'une classification.

Le procédé selon l'invention peut être mis en oeuvre dans des contextes de grandes mouvance des logiques de regroupement ou de classification quand les items de niveau les plus élémentaires sont d'une acception relativement bien partagée. On peut citer à titre d'exemple le contexte de la psychiatrie dans lequel les classifications évoluent beaucoup et où les utilisateurs ne reconnaissent que très rarement ces dernières sans les modifier. Dans un tel contexte, la description factuelle initiale doit être communiquée selon un standard quasi imposé ou reconnu comme tel par le besoin de communication d'un code de même acception, tout en gardant la possibilité de raisonner selon sa propre classification.

Le procédé selon l'invention peut aussi procurer des avantages significatifs en matière de normalisation des termes et acceptions. La normalisation des termes, au sens de la définition de leurs acceptions, est une exigence importante de la communication, en particulier dans le domaine de la médecine. La compilation de termes et locutions au sein d'items IBL d'un fichier FC, autorise la présence de qualifiants complémentaires tels que des qualifiants:
1) précisant l'acception de premier degré, comme dans l'exemple de « l'Ulcère » :
   - « Ulcère gastrique», « Ulcère cutané », « Ulcère cornéen », ...
2) implicites, comme dans l'exemple de « Bronchite chronique », qui implique la présence sous-jacente de certains éléments indispensables :
   « Bronchite chronique » implique obligatoirement l'existence de :
      Toux,
      Expectoration extériorisée ou non,
      Toux au moins trois mois par ans depuis 2 ans,
         et
   « Bronchite chronique » peut comprendre aussi, mais facultativement :
      Sibilants,
      Fièvre,
      Réduction du débit expiratoire de pointe, ... .
3) explicites comme indiqué ci-dessous,
   Toux,
   Expectoration extériorisée ou non,
   Toux au moins trois mois par ans depuis 2 ans,
avec éventuellement en plus :
Fièvre,
Réduction du débit expiratoire de pointe, ... .

Le fichier des items IBL du fichier FC peut donner au choix des termes de niveau différents, offerts pourtant à un même niveau d'accès et sans hiérarchie particulière :
...
Asthme
Bronchite aiguë
Bronchite chronique
BPCO
Broncho-Pneumonie Chr. Obstructive
Etat fébrile
Expectoration extériorisée ou non,
Fièvre
...
Insuffisance respiratoire
...
Réduction du débit expiratoire de pointe
Sibilants
Toux
Toux au moins trois mois par ans depuis 2 ans
Ulcère gastrique
Ulcère cutané
Ulcère cornéen
...

Le procédé de gestion peut aussi être appliqué au retraitement d'informations stockées selon un système de traitement devenu obsolète. Une telle situation peut par exemple intervenir dans un contexte d'évolution de l'offre des prestataires à des utilisateurs médecins informatisés. Ceci peut concerner des médecins dont les données stockées antérieurement doivent évoluer par exemple à suite de l'obsolescence de leur logiciel professionnel, mais dont le contenu, et en particulier les dossiers des patients, ne doit pas disparaître mais au contraire s'intégrer dans un système nouveau qui doit les recevoir. Ces données doivent être reprises en conservant l'historique des observations correspondantes et ceci avec la possibilité d'y adjoindre les mêmes caractéristiques de liens et de fonctionnalités.

Est donné ci-après sous la forme d'un tableau un exemple non limitatif d'une structure de fichier géré par le procédé selon l'invention, comprenant un ensemble d'items à chacun desquels est associé un code DFCC, une information de fréquence et deux codes appartenant respectivement à la 9^{ème} révision et à la 10^{ème} révision de la classification CIM de l'OMS.

| Libelle fichier FC | Code DFCC | Fréquence | Code CIM 9 | Code CIM 10 |
|---|---|---|---|---|
| .... | | | | |
| ASTHME | 5503 | 5552 | 493-90 | J45.9 |
| ASTHME A DYSPNEE CONTINUE | 5542 | 4 | 493-97 | J46.0 |
| ASTHME AIGU | 5539 | 3 | 493-96 | J46.0 |
| ASTHME ALLERGIQUE | 5529 | 660 | 493-93 | J45.0 |
| ASTHME ALLERGIQUE ACARIENS | 22498 | 26 | 493-0 | J45.0 |
| ASTHME ANCIEN | 5504 | 17 | 493-90 | J45.9 |
| ASTHME ATOPIQUE | 5492 | 4 | 493-0 | J45.0 |
| ASTHME BRONCHIQUE | 5525 | 82 | 493-92 | J45.9 |
| ASTHME CARDIAQUE | 4337 | 9 | 428-11 | I50.1 |
| ASTHME CHRONIQUE | 5505 | 48 | 493-90 | J45.9 |
| ASTHME CORTICO-DEPENDANT | 5549 | 25 | 493-99 | J45.9 |
| ASTHME CORTISONE | 17146 | 12 | 493-99 | J45.9 |
| ASTHME DECOMPENSE | 5540 | 1 | 493-96 | J46.0 |
| ASTHME EFFORT | 5506 | 36 | 493-90 | J45.9 |
| ASTHME EXTRINSEQUE | 5495 | 2 | 493-0 | J45.0 |
| ASTHME GRAVE | 15779 | 22 | 493-90 | J46.0 |
| ASTHME HYPEREOSINOPHILE | 21120 | 2 | 493-90 | J82.0 |
| ASTHME INFANTILE | 5496 | 65 | 493-0 | J45.0 |
| ASTHME INFECTIEUX | 5545 | 32 | 493-98 | J45.9 |
| ASTHME INTRINSEQUE | 5501 | 16 | 493-1 | J45.1 |
| ASTHME MODERE | 13847 | 11 | 493-90 | J45.9 |
| ASTHME PAROXYSTIQUE | 5514 | 2 | 493-90 | J45.9 |
| ASTHME POLLINIQUE . | 5497 | 7 | 493-0 | J45.0 |
| ASTHME PROFESSIONNEL | 5531 | 5 | 493-93 | J45.0 |
| ASTHME SEVERE | 5515 | 97 | 493-90 | J46.0 |
| ASTHME SURINFECTE | 5546 | 176 | 493-98 | J45.9 |
| ASTHME TARDIF | 5502 | 12 | 493-1 | J45.9 |
| ATC ASTHME | 10863 | 3 | V12-6 | Z87.0 |
| BPCO ASTHMATIFORME | 5556 | 11 | 496-01 | J44.8 |
| BRONCHITE AIGUE ASTHMATIFORME | 16710 | 3 | 490-04 | J45.9 |
| BRONCHITE ASTHMATIFORME | 5422 | 1420 | 490-04 | J45.9 |
| BRONCHITE ASTHMATIFORME A REPETITION | 5423 | 6 | 490-04 | J45.9 |
| BRONCHITE ASTHMATIFORME ALLERGIQUE | 24489 | 5 | 493-95 | J45.0 |
| BRONCHITE ASTHMATIQUE | 5535 | 171 | 493-94 | J45.9 |
| BRONCHITE CHRONIQUE ASTHMATIFORME | 12174 | 25 | 491-21 | J44.8 |
| BRONCHOPATHIE ASTHMATIFORME | 5428 | 10 | 490-04 | J45.9 |
| CRISE ASTHME | 5541 | 192 | 493-96 | J46.0 |
| ..... | | | | |

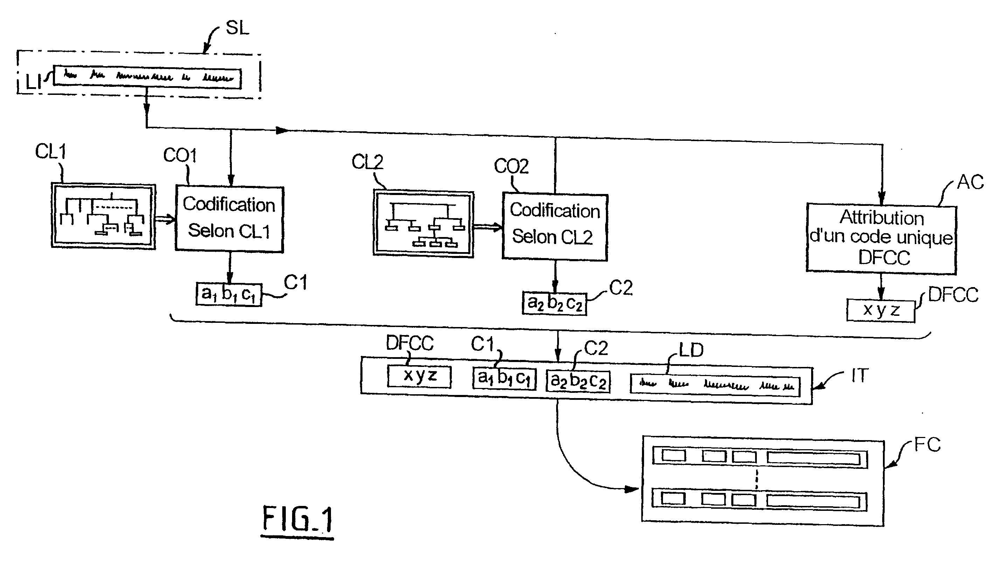

On va maintenant décrire, en référence à la figure 1, des étapes du procédé de génération et d'utilisation d'un fichier d'items désigné dans la suite sous le terme de fichier de codification FC, dans le domaine de l'information médicale.

Un fichier de codification FC selon l'invention est constitué par une accumulation de libellés de diagnostics librement rédigés en langage naturel par des médecins utilisant leurs propres termes. Ces libellés peuvent avoir été saisis à l'issue d'entretiens, d'enquêtes, de campagnes de collecte ou directement par les médecins dans leur pratique quotidienne. Les libellés descriptifs constituent les items du fichier de codification FC. A titre d'exemple non limitatifs, on peut rencontrer les items suivants:
- CARCINOME SPINOCELLULAIRE LEVRE
- TUMEUR MALIGNE LEVRE
- TUMEUR MALIGNE BASE LANGUE
- TUMEUR MALIGNE GLANDE PAROTIDE
etc...

Il est important de noter que les différents items ou libellés peuvent être accumulés séquentiellement, sans filtrage correction ou rangement préalable.

Pour chaque libellé LI ayant fait l'objet d'une saisie SL, est effectué une première opération CO1 de codification selon un premier système de classification CL1,- par exemple la 9ème révision de l'OMS (Organisation Mondiale de la Santé) -, et une seconde opération CO2 de classification selon un second système de classification CL2,- par exemple la 10ème révision de l'OMS. Ces deux opérations de codification conduisent à l'attribution de deux codes de classification C1, C2 pour l'item concerné. Par ailleurs, une opération AC d'attribution d'une code unique DFCC à l'item concerné est effectuée. Ce code unique, désigné à titre d'exemple non limitatif sous le terme de « Diagnosis Fixed Continuity Code (DFCC) », est de préférence attribué séquentiellement. Mais on peut bien sûr dans le cadre de la présente prévoir d'autres formes d'attribution du code unique.

A l'issue de ces opérations de codification et d'attribution, on dispose d'une suite d'items pourvus de code de classification et d'un code DFCC qui constituent le fichier de codification FC.

Ce fichier de codification peut bien sûr faire l'objet de mises à jour par entrée de nouveaux items ou de nouvelles codifications en fonction de l'évolution des systèmes de classification dans le domaine concerné.

Il est à noter que le fichier de codification FC, qui est initialement offert au choix, possède des caractéristiques issues des modalités de sa constitution. On peut y accéder par l'item lui-même ou par usage du code DFCC. Ces caractéristiques sont entre autres des caractéristiques de fréquence ou d'origine, et permettent par exemple:
- d'utiliser ou de constituer des tables de fréquences et d'origines de ces items,
- de moduler l'usage du fichier en fonction des données de fréquence et d'origine,
- de moduler l'usage des tables en transcodage par ces notions de fréquences en cas de relation de type "1:n" entre codes de classifications CL1 et CL2.

A titre d'exemple non limitatif, on donne ci-après un extrait d'un fichier de codification:

| DFCC | Classification 1 | Classification 2 | Libellé Diagnostic |
|---|---|---|---|
| ... | ... | ... | ... |
| 596 | 140-9 | C00.9 | CARCINOME SPINOCELLULAIRE LEVRE |
| 597 | 140-9 | C00.9 | TUMEUR MALIGNE LEVRE |
| 598 | 141-0 | C01.0 | TUMEUR MALIGNE BASE LANGUE |
| 599 | 141-9 | C02.9 | TUMEUR MALIGNE LANGUE |
| ... | | ... | ... |

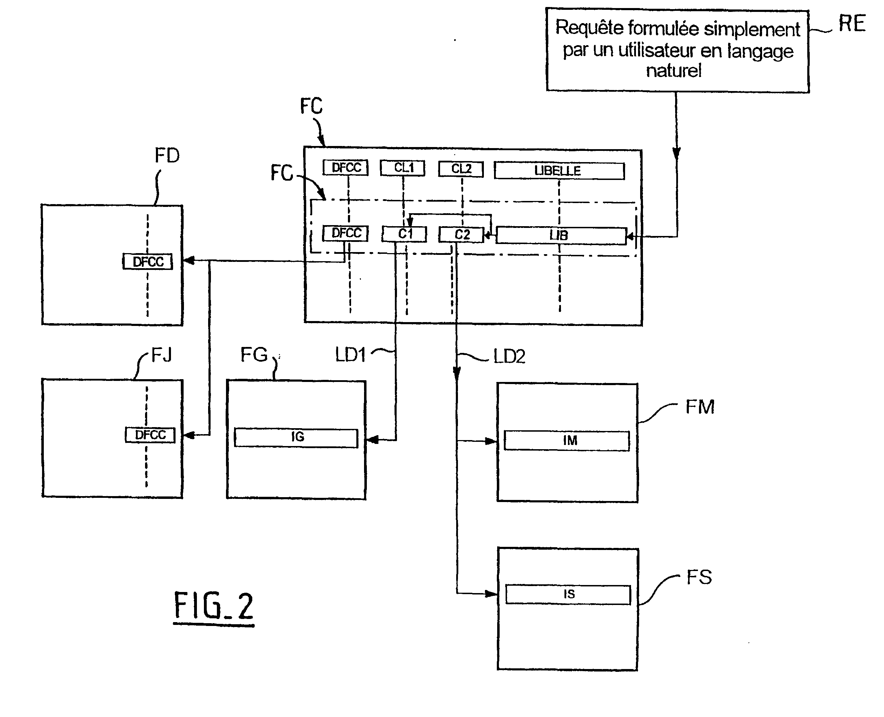

On va maintenant décrire un mode d'utilisation d'un fichier de codification FC par un médecin dans son exercice, en référence à la figure 2. On considère que ce médecin dispose d'un micro-ordinateur dans lequel sont installés un ensemble de logiciels et utilitaires, tels qu'un logiciel de gestion de son cabinet, un logiciel de communication avec des organismes de sécurité sociale et de mutuelle, un logiciel d'aide au diagnostic, ou encore un logiciel d'aide à la prescription de médicaments. Certains de ces logiciels ou utilitaires font référence à des codes de classification de diagnostic et parfois requièrent la saisie de ces codes. Grâce au fichier de codification obtenu avec le procédé selon l'invention, un médecin utilisateur de ces logiciels n'est pas tenu de connaître les différents systèmes de classification, puisque l'utilisateur du fichier de codification permet de générer des liens dynamiques avec des fonctionnalités requérant des codes de classification.

En effet, le médecin accède au fichier de codification et entre une requête exprimée en langage naturel, par exemple, « Tumeur maligne sur une lèvre ». Une procédure de recherche automatisée lui permet d'accéder directement à l'item le plus proche de sa requête. Mais on peut également prévoir que cette recherche soit réalisée à partir de mots-clés suivant des techniques classiques utilisées dans les moteurs de recherche.

Dès qu'un item approprié sous la forme d'un libellé LIB a été identifié au sein du fichier de codification, les codes de classification C1 et C2 correspondant respectivement aux deux systèmes de classification CL1, CL2 permettent d'activer des liens dynamiques LD1, LD2 avec des fonctionnalités disponibles à partir du micro-ordinateur du médecin. Par exemple, le code de classification C2 peut automatiquement activer un lien dynamique avec une base de données scientifiques FS pour fournir des informations scientifiques et médicales IS correspondant au diagnostic objet de la requête et avec une base de données pharmaceutiques FM pour fournir des informations IM sur des spécialités pharmaceutiques de référence, tandis que l'autre code de classification C1 procure un lien dynamique avec une fonctionnalité de gestion FG pour fournir automatiquement à cette fonctionnalité une information IG requérant spécifiquement un code de classification correspondant au premier système de classification. De plus, on peut prévoir une relation externe via le code DFCC ou via l'item lui-même avec des fonctions de tables de décomptage FD ou des fonctions de mise à jour FJ.

Par ailleurs, à partir d'un fichier de codification FC initial offert au choix, l'utilisateur peut simplement sélectionner des items répondant à ses attentes, par exemple relatifs à un patient donné, et constituer ainsi un fichier FR résultant de ses choix. Il peut alors utiliser ce fichier résultat FR issu d'opérations de choix d'items CI, qui représente ainsi un extrait du fichier initial FC offert au choix, dans les mêmes conditions que ce dernier.

Il est à noter que les fonctionnalités du fichier de codification généré avec le procédé selon l'invention s'inscrivent dans un contexte de relation, par exemple de type bases de données relationnelles et mettent en oeuvre les outils informatiques classiquement utilisés dans ce contexte.

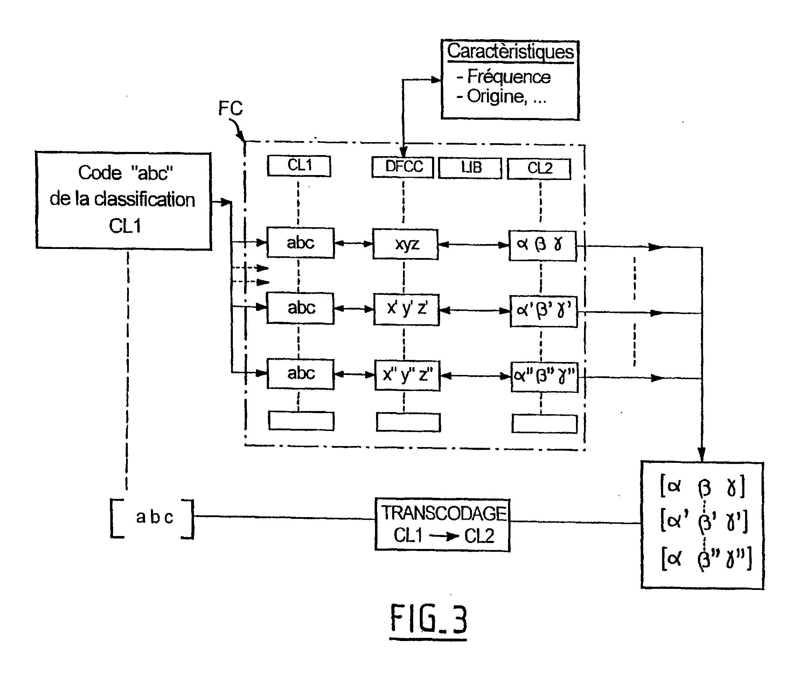

Le procédé selon l'invention peut trouver également une application avantageuse en matière de transcodage entre deux systèmes de classification coexistants, comme l'illustre la figure 3. Ainsi, à partir d'un code "abc" appartenant à un premier système de classification CL1, il est possible d'identifier l'ensemble des items du fichier de codification FC auxquels ce premier code "abc" a été attribué. Ces items identifiés ont chacun un code DFCC unique xyz,..,x'y'z',..,x''y''z'' et sont également affectés chacun d'un code de classification selon le second système de classification CL2. Ces codes de classification αβγ,.., α'β'γ',.., α''β''γ'' constituent le résultat de l'opération de transcodage effectué sur le code abc initial. Cette opération élémentaire peut être automatisée pour fournir un transcodage complet entre les deux systèmes de classification. Ce transcodage est rendu possible grâce à la structure spécifique du fichier de codification. Par ailleurs, on peut avantageusement prévoir que l'utilisation du fichier à des fins de transcodage soit modulée de fonction de caractéristiques de fréquence et d'origine des items, accessibles via le code DFCC, notamment pour permettre des allocations proportionnelles dans des relations de type "1:n".

Pour la réalisation d'un système de génération d'un fichier de codification selon l'invention, on peut mettre en oeuvre des moyens classiques de saisie, de traitement, de stockage et de recherche d'informations. Les techniques mises en oeuvre dans la maintenance et la gestion des bases de données relationnelles peuvent naturellement être mises à contribution.

Bien sûr, l'invention n'est pas limitée aux exemples qui viennent d'être décrits et de nombreux aménagements peuvent être apportés à ces exemples sans sortir du cadre de l'invention. En particulier, le nombre de classifications utilisées n'est pas limité à deux et d'autres techniques d'attribution du code unique DFCC peuvent être envisagées. Par ailleurs, la présente invention peut trouver des applications dans d'autres domaines des sciences et techniques de l'informations, notamment en gestion électronique de documents.

## Revendications

1. Procédé pour gérer et utiliser un fichier (FC) d'items (IBL) dans un espace d'informations, notamment d'informations médicales, stockées dans au moins une base de données et pouvant être classées selon une pluralité de systèmes de classification (CL1, CL2), ce fichier d'items (FC) étant généré par accumulation d'items rédigés dans un langage dont le langage naturel,
ce procédé comprenant une attribution à chaque item (IBL), en plus de ses caractéristiques propres, de codes de classification (C1, C2) appartenant chacun à un système de classification (CL1, CL2) parmi la pluralité de systèmes de classification, de sorte que les codes de classification ainsi attribués procurent des liens (LD1, LD2) avec d'autres fonctionnalités ou bases de données,
**caractérisé en ce qu'**il comprend en outre les étapes consistant à :
- affecter à chaque item (IBL) la propriété de constituer la plus petite partie commune entre différents codes de classification attribués,
- attribuer à chaque item (IBL) constituant la plus petite partie commune entre les différents codes de classification attribués audit item, un code unique (DFCC) qui lui est propre, et
- détecter ceux des items (IBL) issus du fichier d'items, qui sont liés à « n » codes de « n » systèmes de classification et qui sont le lieu d'une relation bijective entre les codes de ces « n » systèmes de classification dont lesdits items sont l'intersection.

2. Procédé selon la revendication 1, **caractérisé en ce qu'**il est en outre agencé pour procurer un transcodage entre un premier système de classification (CL1) et un second système de classification (CL2) parmi la pluralité de systèmes de classification via le fichier d'items (FC) généré avec ce procédé.

3. Procédé selon la revendication 2, **caractérisé en ce que** ce transcodage prend en compte des caractéristiques des items, notamment des caractéristiques de fréquence, accessibles via le code unique (DFCC)ou via l'item (IBL).

4. Procédé selon l'une quelconque des revendications précédentes, **caractérisé en ce que** des items du fichier d'items revêtent la nature de terme ou locution de type nomenclature, chacun de ces items constituant un élément commun d'intersection de l'acception de deux codes de deux classifications différentes.

5. Procédé selon l'une quelconque des revendications précédentes, **caractérisé en ce qu'**il comprend en outre un traitement de caractéristiques, notamment de fréquence, d'items (IBL) du fichier (FC) d'items pour pondérer des observations réelles des items constituant la plus petite partie commune entre plusieurs systèmes de classifications.

6. Procédé selon l'une quelconque des revendications précédentes, **caractérisé en ce que** le code unique (DFCC) attaché à chaque item au sein du fichier d'items est attribué séquentiellement.

7. Procédé selon l'une quelconque des revendications précédentes, **caractérisé en ce que** le code unique (DFCC) est agencé pour procurer lui aussi un lien simple avec d'autres caractéristiques de l'item (IBL), notamment des caractéristiques d'observations antérieures.

8. Procédé selon l'une quelconque des revendications précédentes, **caractérisé en ce qu'**il comprend en outre une sélection, au sein du fichier d'items (FC), d'items répondant aux attentes d'un utilisateur, de façon à constituer un fichier résultat (FR) extrait du fichier d'items (FC) et pourvu des mêmes caractéristiques de lien avec des bases et fonctionnalités extérieures.

9. Procédé selon l'une des revendications précédentes, **caractérisé en ce qu'**il est agencé pour générer, pour chaque ligne du fichier d'items (IBL) , un libellé (LIB) pouvant être rédigé en langage naturel et constituant l'item, un code unique (DFCC) attaché à ce libellé, et plusieurs codes (C1, C2) issus de plusieurs classifications (CL1, CL2).

10. Système pour gérer et utiliser un fichier (FC) d'items (IBL) dans un espace d'informations, notamment d'informations médicales, stockées dans au moins une base de données et pouvant être classées selon une pluralité de systèmes de classification (CL1, CL2), mettant en oeuvre le procédé selon l'une quelconque des revendications précédentes,
ce système comprenant :
- des moyens pour accumuler au sein de ce fichier d'items (FC) des items rédigés dans un langage tel que le langage naturel par des utilisateurs de cette base de données, et des moyens pour attribuer à chaque item au sein dudit fichier d'items (FC) plusieurs codes de classification (C1, C2) appartenant chacun à un système de classification (CL1, CL2) parmi la pluralité de systèmes de classification, de sorte que les codes de classification (C1, C2) ainsi attribués procurent des liens avec d'autres fonctionnalités ou bases de données,
**caractérisé en ce qu'**il comprend en outre :
- des moyens pour attribuer à chaque item constituant la plus petite partie commune entre les différents codes de classification attribués audit item, un code unique (DFCC) qui lui est propre,
- des moyens pour associer à chaque code de classification appartenant à un système de classification, les items auxquels ledit code de classification a été attribué, et
- des moyens pour identifier ceux des items issus du fichier d'items, qui sont liés à « n» codes de « n » systèmes de classification et sont le lieu d'une relation bijective entre les codes de deux systèmes de classification.

11. Système selon la revendication 10, **caractérisé en ce qu'**il comprend en outre des moyens pour réaliser un transcodage entre un premier système de classification (CL1) et un second système de classification (CL2) parmi la pluralité de systèmes de classification via le fichier de codification (FC) généré avec ce procédé.

12. Application du procédé selon l'une des revendications 1 à 9 à un système d'information médicale, dans laquelle chaque item (IBL) consiste en un libellé de maladies, diagnostics ou raisons de consultation médicale.

13. Application du procédé selon l'une quelconque des revendications 1 à 9, à un système d'information, notamment un système d'information médicale, **caractérisée en ce que** le fichier de codification procure notamment un accès à des bases de données et fonctionnalités qui utilisent ou contiennent l'une au moins des classifications utilisées dans ledit fichier de codification ou dans un fichier résultant d'un choix effectué au sein dudit fichier de codification.

## Patentansprüche

1. Verfahren zur Verwaltung und Verwendung einer Datei (FC) von Datensätzen (IBL) in einem Informationsraum, insbesondere in einem Raum von medizinischen Informationen, die in mindestens einer Datenbank gespeichert sind und nach einer Vielzahl von Klassifizierungssystemen (CL1, CL2) geordnet sein können, wobei diese Datensatzdatei (FC) durch Akkumulation von Datensätzen erzeugt wird, die in einer Sprache, insbesondere in der natürlichen Sprache, abgefaßt sind, wobei dieses Verfahren umfaßt, daß jedem Datensatz (IBL) zusätzlich zu seinen eigenen Merkmalen Klassifizierungscodes (C1, C2) zugeteilt werden, die jeweils zu einem Klassifizierungssystem (CL1, CL2) aus der Vielzahl von Klassifizierungssystemen gehören, so daß die auf diese Weise zugeteilten Klassifizierungscodes Verknüpfungen (LD1, LD2) mit anderen Funktionalitäten oder Datenbanken bilden,
**dadurch gekennzeichnet, daß** es außerdem die folgenden Schritte umfaßt, die darin bestehen,
- daß jedem Datensatz (IBL) die Eigenschaft zugeteilt wird, den kleinsten gemeinsamen Teil zwischen verschiedenen zugeteilten Klassifizierungscodes zu bilden,
- daß jedem Datensatz (IBL), der den kleinsten gemeinsamen Teil zwischen den verschiedenen, diesem Datensatz zugeordneten Klassifizierungscodes bildet, ein einziger, für ihn spezifischer Code (DFCC) zugeteilt wird, und
- daß diejenigen der aus der Datensatzdatei stammenden Datensätze (IBL) erfaßt werden, die mit "n" Codes von "n" Klassifizierungssystemen verknüpft sind und die der Ort einer bijektiven Beziehung zwischen den Codes dieser "n" Klassifizierungssysteme sind, deren Konjunktion diese Datensätze sind.

2. Verfahren nach Anspruch 1, **dadurch gekennzeichnet, daß** es außerdem dafür ausgelegt ist, eine Umcodierung zwischen einem ersten Klassifizierungssystem (CL1) und einem zweiten Klassifizierungssystem (CL2) aus der Vielzahl von Klassifizierungssystemen über die mit diesem Verfahren erzeugte Datensatzdatei (FC) vorzunehmen.

3. Verfahren nach Anspruch 2, **dadurch gekennzeichnet, daß** diese Umcodierung Merkmale der Datensätze, insbesondere Frequenzmerkmale, berücksichtigt, die über den einzigen Code (DFCC) oder über den Datensatz (IBL) zugänglich sind.

4. Verfahren nach einem der vorhergehenden Ansprüche, **dadurch gekennzeichnet, daß** Datensätze der Datensatzdatei eine Begriffs- oder Ausdrucksnatur vom Typ Nomenklatur besitzen, wobei jeder dieser Datensätze ein gemeinsames Element der Konjunktion der Bedeutung von zwei Codes von zwei verschiedenen Klassifizierungen bildet.

5. Verfahren nach einem der vorhergehenden Ansprüche, **dadurch gekennzeichnet, daß** es außerdem eine Verarbeitung von Merkmalen, insbesondere Frequenzmerkmalen, von Datensätzen (IBL) der Datensatzdatei (FC) umfaßt, um tatsächliche Beobachtungen der den kleinsten gemeinsamen Teil zwischen mehreren Klassifizierungssystemen bildenden Datensätze zu gewichten.

6. Verfahren nach einem der vorhergehenden Ansprüche, **dadurch gekennzeichnet, daß** der mit jedem Datensatz in der Datensatzdatei verbundene einzige Code (DFCC) sequenziell zugeteilt wird.

7. Verfahren nach einem der vorhergehenden Ansprüche, **dadurch gekennzeichnet, daß** der einzige Code (DFCC) ausgelegt ist, um seinerseits auch eine einfache Verknüpfung mit anderen Merkmalen des Datensatzes (IBL), insbesondere Merkmale von früheren Beobachtungen, zu bilden.

8. Verfahren nach einem der vorhergehenden Ansprüche, **dadurch gekennzeichnet, daß** es außerdem umfaßt, daß in der Datensatzdatei (FC) Datensätze ausgewählt werden, die den Erwartungen eines Benutzers entsprechen, so daß eine aus der Datensatzdatei (FC) extrahierte Ergebnisdatei (FR) gebildet wird, die mit denselben Merkmalen der Verknüpfung mit Banken und äußeren Funktionalitäten versehen ist.

9. Verfahren nach einem der vorhergehenden Ansprüche, **dadurch gekennzeichnet, daß** es ausgelegt ist, um bei jeder Zeile der Datensatzdatei (IBL) eine Beschreibung (LIB), die in natürlicher Sprache abgefaßt sein kann und den Datensatz bildet, einen mit dieser Beschreibung verknüpften einzigen Code (DFCC) und mehrere, von mehreren Klassifizierungen (CL1, CL2) stammende Codes (C1, C2) zu erzeugen.

10. System zur Erzeugung und Verwendung einer Datei (FC) von Datensätzen (IBL) in einem Informationsraum, insbesondere in einem Raum von medizinischen Informationen, die in mindestens einer Datenbank gespeichert sind und nach einer Vielzahl von Klassifizierungssystemen (CL1, CL2) geordnet werden können, unter Verwendung des Verfahrens nach einem der vorhergehenden Ansprüche,
umfassend:
- Mittel, um in dieser Datensatzdatei (FC) Datensätze, die in einer Sprache wie der natürlichen Sprache abgefaßt sind, durch Benutzer dieser Datenbank zu akkumulieren, und Mittel, um jedem Datensatz in dieser Datensatzdatei (FC) mehrere Klassifizierungscodes (C1, C2) zuzuteilen, die jeweils zu einem Klassifizierungssystem (CL1, CL2) aus der Vielzahl von Klassifizierungssystemen gehören, so daß die auf diese Weise zugeteilten Klassifizierungscodes (C1, C2) Verknüpfungen mit anderen Funktionalitäten oder Datenbanken bilden,
**dadurch gekennzeichnet, daß** es außerdem umfaßt:
- Mittel, um jedem Datensatz, der den kleinsten gemeinsamen Teil zwischen den verschiedenen diesem Datensatz zugeteilten Klassifizierungscodes bildet, einen einzigen für ihn spezifischen Code (DFCC) zuzuteilen,
- Mittel, um jedem zu einem Klassifizierungssystem gehörenden Klassifizierungscode die Datensätze zuzuordnen, denen dieser Klassifizierungscode zugeteilt wurde, und
- Mittel, um diejenigen der von der Datensatzdatei stammenden Datensätze zu identifizieren, die mit "n" Codes von "n" Klassifizierungssystemen verknüpft sind und der Ort einer bijektiven Beziehung zwischen den Codes von zwei Klassifizierungssystemen sind.

11. System nach Anspruch 10, **dadurch gekennzeichnet, daß** es außerdem Mittel zur Durchführung einer Umcodierung zwischen einem ersten Klassifizierungssystem (CL1) und einem zweiten Klassifizierungssystem (CL2) aus der Vielzahl von Klassifizierungssystemen über die mit diesem Verfahren erzeugte Codierungsdatei (FC) umfaßt.

12. Anwendung des Verfahrens nach einem der Ansprüche 1 bis 9 auf ein medizinisches Informationssystem, bei der jeder Datensatz (IBL) aus einer Beschreibung von Krankheiten, Diagnosen oder Gründen der ärztlichen Konsultation besteht.

13. Anwendung des Verfahrens nach einem der Ansprüche 1 bis 9 auf ein Informationssystem, insbesondere ein medizinisches Informationssystem, **dadurch gekennzeichnet, daß** die Codierungsdatei insbesondere einen Zugang zu Datenbanken und Funktionalitäten liefert, die mindestens eine der Klassifizierungen verwenden oder enthalten, die in dieser Codierungsdatei oder in einer Datei verwendet werden, die sich aus einer in dieser Codierungsdatei durchgeführten Wahl ergibt.

## Claims

1. Method for managing and using an item (IBL) file (PC) in an information space, particularly a medical information space, stored in at least one database and being able to be stored according to a plurality of classification systems(CL1, CL2), this item file (FC) being generated by accumulating items written in a language including natural language,
this method involving an assignment to each item (IBL), in addition to its own characteristics, of several classification codes (C1, C2) each belonging to a classification system (CL1, CL2) among the plurality of classification systems, such that the classification codes thus assigned provide links (LD1, LD2) to other functions or databases,
**characterized in that** it further comprises steps consisting in:
- assigning to each item (IBL) the property of constituting the smallest common part between different classification codes assigned,
- assigning to each item (IBL) constituting the smallest common part between the different classification codes assigned to said item a unique code (DFCC) which is specific to it, and
- detecting those items (IBL) originating from the item file which are linked to "n" codes of "n" classification systems and which are the location of a bijective relationship between the codes of these "n" classification systems of which said items are the intersection.

2. Method according to claim 1, **characterized in that** it is further designed to provide transcoding between a first classification system (CL1) and a second classification system (CL2) from among the plurality of classification systems via the item file (FC) generated with this method.

3. Method according to claim 2, **characterized in that** the transcoding takes account of the characteristics of the items, in particular the frequency characteristics, accessible via the unique code (DFCC) or via the item (IBL).

4. Method according to any one of the previous claims, **characterized in that** items in the item file assume the character of term or nomenclature type locution, each of these items constituting a common intersection element of the meaning of two codes of two different classifications.

5. Method according to any one of the previous claims, **characterized in that** it further comprises a processing of characteristics, in particular frequency characteristics, of items (IBL) in the item file (PC) in order to weight real observations of items constituting the smallest common element between several classification systems.

6. Method according to any one of the previous claims, **characterized in that** the unique code (DFCC) attached to each item in the item file is assigned sequentially.

7. Method according to any one of the previous claims, **characterized in that** the unique code (DFCC) is designed to also give a simple link to other characteristics of the item (IBL), in particular the characteristics of earlier observations.

8. Method according to any one of the previous claims, **characterized in that** it further comprises a selection, within the item file (FC), of items meeting the user's expectations, in order to constitute a result file (FR) extracted from the item file (FC) and endowed with the same link characteristics to external databases and functions.

9. Method according to any one of the previous claims, **characterized in that** it is designed to generate, for each line in the item (IBL) file, a text (LIB) which can be written in natural language and constituting the item, a unique code (DFCC) attached to this text and several codes (C1, C2) originating from several classifications (CL1, CL2).

10. System for managing and using an item (IBL) file (FC) in an information space, particularly a medical information space, stored in at least one database and being able to be classified according to a plurality of classification systems (CL1, CL2), implementing the method according to any one of the previous claims, this system comprising:
- means for accumulating, within this item file (FC), items written in a language such as natural language by the users of this database, and means for assigning to each item within said item file (FC) several classification codes (C1, C2) each belonging to a classification system (CL1, CL2) among the plurality of classification systems, such that the classification codes (C1, C2) assigned in this way provide links to other functions or databases, **characterized in that** it further comprises:
- means for assigning a unique specific code (DFCC) to each item constituting the smallest common part between the different classification codes assigned to said item,
- means for associating with each classification code belonging to a classification system the items to which said classification code has been assigned, and
- means for identifying those items originating from the item file which are linked to "n" classification codes of "n" classification systems and are the location of a bijective relationship between the codes of two classification systems.

11. System according to claim 10, **characterized in that** it further comprises means for realizing a transcoding between a first classification system (CL1) and a second classification system (CL2) among the plurality of classification systems via the coding file (FC) generated with this method.

12. Application of the method according to one of claims 1 to 9 to a medical information system in which each item (IBL) consists of a text listing diseases, diagnoses or reasons for medical consultation.

13. Application of the method according to any one of claims 1 to 9 to an information system, in particular a medical information system, **characterized in that** the coding file provides in particular access to databases and functions which use or contain one at least of the classifications used in said coding file or in a file resulting from a choice made within said coding file.
